# EUROPEAN PATENT SPECIFICATION

(11) **EP 3 459 513 B1**
(45) Date of publication and mention of the grant of the patent: **08.04.2020**
(21) Application number: 17823899.4
(22) Date of filing: 26.05.2017
(51) Int. Cl.: A61F 13/511, A61F 13/53, A61F 13/532, A61F 13/533, A61F 13/513, A61F 13/535, A61F 13/84, A61F 13/536, A61F 13/539

(54) **ABSORBENT ARTICLE**
SAUGFÄHIGER ARTIKEL
ARTICLE ABSORBANT

(30) Priority: 07.07.2016 JP 2016135125
(43) Date of publication of application: 27.03.2019
(73) Proprietor: Unicharm Corporation, Ehime 799-0111 (JP)
(72) Inventor: GODA, Hiroki, Kanonji-shi Kagawa 769-1602 (JP); YAMAGUCHI, Masashi, Kanonji-shi Kagawa 769-1602 (JP); TANIGUCHI, Hiroaki, Kanonji-shi Kagawa 769-1602 (JP)
(74) Representative: Dolleymores
(86) International application number: PCT/JP2017/019789
(87) International publication number: WO 2018/008292

(56) References cited:
- EP-A1- 2 248 497
- EP-A1- 2 886 093
- EP-A2- 2 246 021
- JP-A- 2008 272 269
- JP-A- 2009 207 684
- JP-A- 2014 200 372
- JP-A- 2016 028 785
- US-A1- 2007 087 169

## Description

### FIELD

The present invention relates to an absorbent article such as a disposable diaper, incontinence pad or sanitary napkin, and especially to an absorbent article having coloration in recesses formed in an absorbent core.

### BACKGROUND

PTL 1 (Japanese Unexamined Patent Publication No. 2015-533623) discloses an absorbent article having, in the absorbent core of the absorbent article, a permanent channel that does not include super-absorbent polymer particles, and having a printed adhesive layer indicating the channel between the top sheet and the absorbent core, in order to communicate to the caregiver that the channel exists for more rapid and efficient liquid absorption. It also teaches that the absorbent article may further include embossing to indicate the channel.

PTL 2 (Japanese Unexamined Patent Publication No. 2006-181193) discloses an absorbent article having a plurality of recesses, depressed in the thickness direction, formed on the skin contact surface side of an absorbing body in regions further outward than the absorbent body in the planar view, for the purpose of increasing the leakproofness and also helping the wearer be aware of the increased leakproofness to provide a feeling of assurance, and provided with printed sections created by transfer of ink on the bottom parts of the recesses, forming a design.

EP2248497A1 discloses an absorbent article having a recess part, at least a portion of which has a colored layer in contact with the back surface of a liquid-permeable sheet.

EP2246021A2 discloses an absorbent article including a plurality of longitudinally-extending channels in a central region; the channels are provided with color in one embodiment.

EP2886093A1 relates to an absorbent article where at least a portion of the top side of a core wrap is colored to form one or more colored areas visible from the exterior of the absorbent article.

### [CITATION LIST]

### [PATENT LITERATURE]

[PTL 1] Japanese Unexamined Patent Publication No. 2015-533623
[PTL 2] Japanese Unexamined Patent Publication No. 2006-181193

### SUMMARY

### [TECHNICAL PROBLEM]

In PTL 1, embossing is also utilized in addition to the printed adhesive layer that indicates the channel, and while the stamped grooves formed by embossing appear to be of a different color than the non-colored sections that are not the stamped grooves, the coloration of the stamped grooves is coloration in an integral fashion with coloration of the other pattern created by printing, and since the integral coloration results in the same color in both the stamped grooves and the other regions, it has not been possible to display the stamped grooves in a more distinct manner.

In PTL 2, the recesses are formed in regions further outward than the absorbent body, and are therefore less than satisfactory in terms of appeal of the structure and function of the absorbent body for the wearer, while there is only a solid coloration at the bottom parts of the recesses, and no modifications are made to render the recesses more clearly visible in three dimensions.

It is therefore an object of the present invention to provide an absorbent article comprising recesses in a liquid-permeable base sheet on the skin side of an absorbent core, and the absorbent core, and having coloration allowing the recess shapes to be more easily seen in three dimensions.

### [SOLUTION TO PROBLEM]

The invention that solves the aforementioned problems includes the following aspects.

(Aspect 1) An absorbent article comprising an absorbent core and a liquid-permeable base sheet on the skin side of the absorbent core, and having a thickness direction, wherein
the base sheet and the absorbent core have recesses extending in the thickness direction from the surface of the skin side of the base sheet up to the absorbent core,
the recesses each have a bottom part and wall sections that rise from the bottom part in a direction toward the surface side of the skin side,
the base sheet has, in the recesses, colored regions that are visible from the skin side, and
the grayscale value (where black is represented by 0 and white is represented by any positive value, same hereunder) of the coloration of each linear region that includes the border line between the bottom part and the wall section and is delineated by a prescribed width is lower than the grayscale value of the coloration in each of the regions delineated by the same width as the prescribed width of the linear region, on both sides adjacent to the linear region, wherein the absorbent article has a core wrap covering the absorbent core, the core wrap has an upper core wrap on the skin side of the absorbent core, the base sheet is the upper core wrap, and at the recesses, the surface of the skin side or the absorbent core side of the upper core wrap is colored with a pressure sensitive coloring agent.

(Function and effect) Since colored regions that are visible from the skin side are present in the recesses running from the surface of the base sheet located on the skin side of the absorbent article up to the interior of the absorbent core, with the colored regions matching the recesses, and in the linear regions including the border lines between the bottom parts and the wall sections of the recesses, the grayscale value of the coloration (where black is represented by 0 and white is represented by any positive value) is lower than regions of same widths as the linear regions on both sides sandwiching the linear regions, the shapes of the recesses (bottom parts) are distinct and the recesses can be clearly seen. Since the recesses can be clearly seen, the user can be made aware that embossing has been formed in the recesses that produce a firm physical feeling in the absorbent body, and that the absorbent body has curved deforming sections, and can also be provided with an actual firm feeling and a reliable feeling of deformation by the curved deforming sections. In addition, because of the difference in the grayscale values of coloration due to the regions in the recesses, the colored regions are more complex than when they have uniform coloration, and they have a more three-dimensional (natural) appearance.

(Aspect 2) The absorbent article according to aspect 1, wherein the grayscale values of coloration in the regions other than the linear regions of the bottom parts are not uniform.

(Function and effect) Since the grayscale values of coloration in the regions other than the linear regions at the bottom parts of the recesses are not uniform (irregularities in concentration exist, with high sections and low sections), the linear regions that have low grayscale values of coloration are accentuated and the recesses (bottom parts) can be more clearly seen. Also, since coloration of the bottom parts is not uniform, the recesses appear undulating and the shapes of the recesses appear more three-dimensional (natural).

(Aspect 3) The absorbent article according to aspect 1 or 2, wherein the recesses have colored regions in both the bottom parts and the wall sections.

(Function and effect) When the wall sections of the recesses are colored, the coloration of the wall sections is fainter coloration than the coloration of the bottom parts because the wall sections are more resistant to coloring, and therefore grayscale value-based shadowing occurs on coloration of the recesses at the bottom parts and wall sections, allowing the recesses to be more clearly seen.

(Aspect 4) The absorbent article according to aspect 3, wherein the colored regions of the wall sections have grayscale values of coloration that are higher as they recede from the linear regions in the thickness direction.

(Function and effect) If the coloration of the wall sections of the recesses has gradation such that the grayscale values of coloration are higher as they recede from the linear regions in the thickness direction of the absorbent body, the recesses appear three-dimensional, and the recesses can be more clearly seen.

(Aspect 5) The absorbent article has a core wrap covering the absorbent core, the core wrap has an upper core wrap on the skin side of the absorbent core, the base sheet is the upper core wrap, and at the recesses, the surface of the skin side of the upper core wrap is colored with a pressure sensitive coloring agent.

(Function and effect) Since the skin side surface of the upper core wrap has coloration, the coloration of the absorbent body is easily to see from the skin side. Since the recesses are colored using a pressure sensitive coloring agent during the step of forming the recesses, the coloration matches the recesses and the recesses can be clearly seen.

(Aspect 6) Alternatively the absorbent article has a core wrap covering the absorbent core, the core wrap has an upper core wrap on the skin side of the absorbent core, the base sheet is the upper core wrap, and at the recesses, the surface of the absorbent core side of the upper core wrap is colored with a pressure sensitive coloring agent.

(Function and effect) Since coloration is accomplished using a pressure sensitive coloring agent during the step of forming the recesses, the coloration matches the recesses and the recesses can be clearly seen. Since the pressure sensitive coloring agent is present on the surface of the absorbent body core side, the excess pressure sensitive coloring agent is rubbed off in the regions that are not to be colored during production and use of the absorbent body, helping to prevent unintended coloration or color migration due to the pressure sensitive coloring agent.

(Aspect 7) The absorbent article according to any one of aspects 1 to 6, wherein the absorbent article has a core wrap covering the absorbent core, the core wrap has a lower core wrap on the non-skin side of the absorbent core and has a second colored region that coincides with the recesses, on the surface of the lower core wrap, and the second colored region is visible from the non-skin side.

(Function and effect) Since coloration coinciding with the recesses is also present in the lower core wrap on the non-skin side of the absorbent body, the recesses are also visible from the non-skin side.

(Aspect 8) The absorbent article according to any one of aspects 5 to 7, wherein the core wrap is tissue.

(Function and effect) Since tissue has high fiber density, the coloration is more distinct and easy to see.

### [ADVANTAGEOUS EFFECTS OF INVENTION]

According to the invention there is provided an absorbent article comprising recesses in a liquid-permeable base sheet on the skin side of an absorbent core, and the absorbent core, and having coloration allowing the recess shapes to be more easily seen in three dimensions.

### BRIEF DESCRIPTION OF DRAWINGS

FIG. 1 is an expanded plan view of a disposable diaper 1 as an embodiment.
FIG. 2 is a plan view of the skin side of the absorbent body 10 of the embodiment.
FIG. 3 is an end view of a cross section of the absorbent body 10 of FIG. 2, along line III-III.
FIG. 4 is a partial magnified perspective view of the absorbent body 10 shown in FIG. 2 where the lattice-like first embossed section PI has been cut along line IV-IV.
FIG. 5 is a partial magnified perspective view of a second embossed section P2 serving as a curving origin (hinge) in the absorbent body 10 shown in FIG. 2.
FIG. 6 is a graph showing grayscale values for an example of an embossed section (recess).
FIG. 7 is a magnified cross-sectional view and a plan view of a raised section 32-1 formed in an embossing roll for formation of a recess 22 as the embossed section shown in FIG. 4.
FIG. 8 is a magnified cross-sectional view and a plan view of a raised section 32-2 formed in a different embossing roll for formation of a recess 22 as the embossed section shown in FIG. 4.
FIG. 9 is an end view of a cross section at the center section in the lengthwise direction of a disposable diaper according to a different embodiment.

### DESCRIPTION OF EMBODIMENTS

The absorbent article of the invention is an absorbent article comprising an absorbent core and a liquid-permeable base sheet on the skin side of the absorbent core, and having a thickness direction, wherein the base sheet and the absorbent core have recesses extending in the thickness direction from the surface of the base sheet up to the absorbent core, the recesses each have a bottom part and wall sections that rise from the bottom part in a direction toward the surface of the skin side of the base sheet, the base sheet has in the recesses colored regions that are visible from the skin side, and the grayscale value of the coloration of each linear region that includes the border line between the bottom part and a wall section and is delineated by a prescribed width is lower than both of the grayscale values of coloration in the regions delineated by the same width as the prescribed width of the linear region, on both sides adjacent to the linear region.

### (Embodiment of disposable diaper)

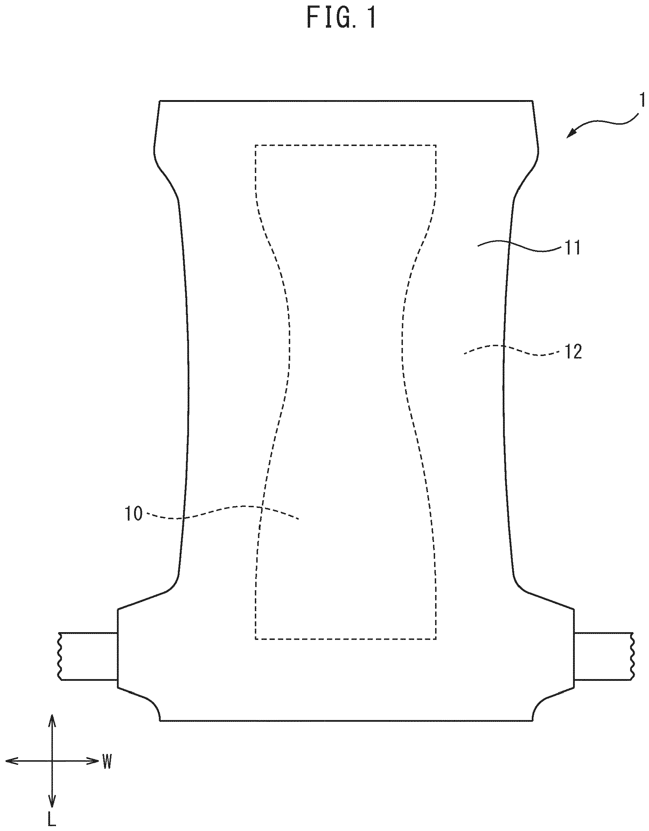

A disposable diaper 1 as an embodiment of the absorbent article of the invention will now be explained with reference to the accompanying drawings. Fig. 1 is an expanded plan view of a disposable diaper 1 as an embodiment.

The disposable diaper 1 has a lengthwise direction L and a widthwise direction W in the plan view of Fig. 1, and also has a thickness direction T in the direction perpendicular to the lengthwise direction L and widthwise direction W.

The absorbent body 10 is located at the center section in the lengthwise direction L and widthwise direction W of the disposable diaper 1, and it has a liquid-permeable top sheet 11 on the skin side of the absorbent body 10 (the front end direction perpendicular to the plane of the plan view in Fig. 1), and a liquid-impermeable back sheet 12 on the opposite side from the skin side (the non-skin side). While not shown, the disposable diaper 1 may also have a second liquid-permeable sheet between the top sheet 11 and the absorbent body 10.

The absorbent body 10 of the disposable diaper of this embodiment will be described below. The top sheet 11 is optically transparent, so that the colored region formed in the absorbent body 10 is visible. The degree of optical transparency may be evaluated by the light transmittance, but in this case it is important for the colored region formed in the absorbent body 10 to be visible. For example, the light transmittance may be 60 to 95% and is preferably 65 to 90%, but since this will depend on the density of coloration of the colored region formed in the absorbent body 10, the light transmittance cannot be specified for all cases. Moreover, the top sheet 11 may be optically transparent, and may also have an opening, with the colored region formed in the absorbent body 10 being visible through the opening.

For embodiments of the invention, the construction of the disposable diaper 1 is not limited to the construction of the embodiment described above, and it may have the construction of any publicly known disposable diaper, so long as the prescribed coloration of the invention is formed in the recesses of the absorbent body.

### (Absorbent body)

The disposable diaper 1 of this embodiment has, in the absorbent body 10, recesses 22 running from the surface of the upper core wrap 10-3 of the core wrap 10-2 that covers the absorbent core 10-1, to the absorbent core 10-1, and has colored regions in the recesses 22. The absorbent body 10 will now be described.

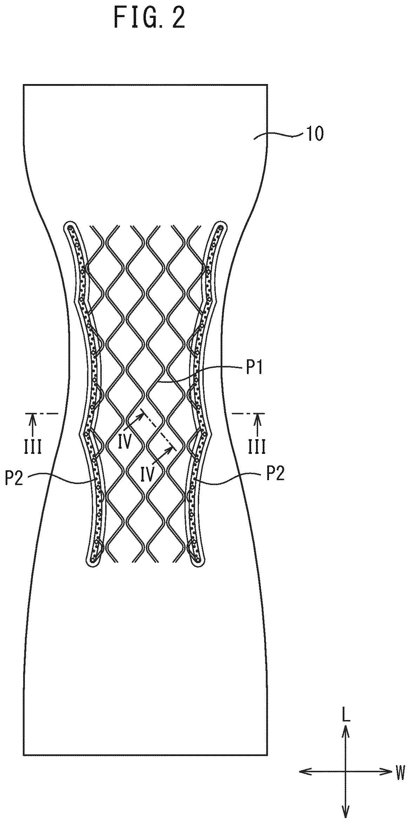

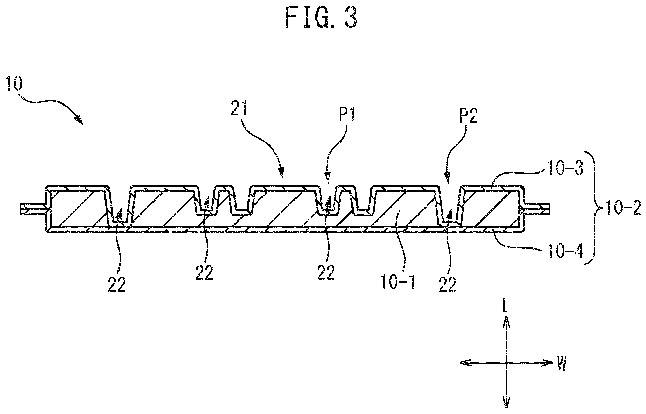

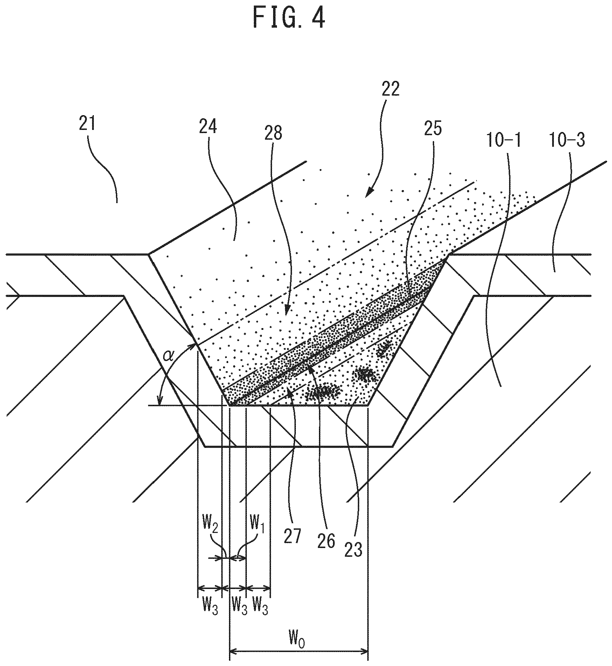

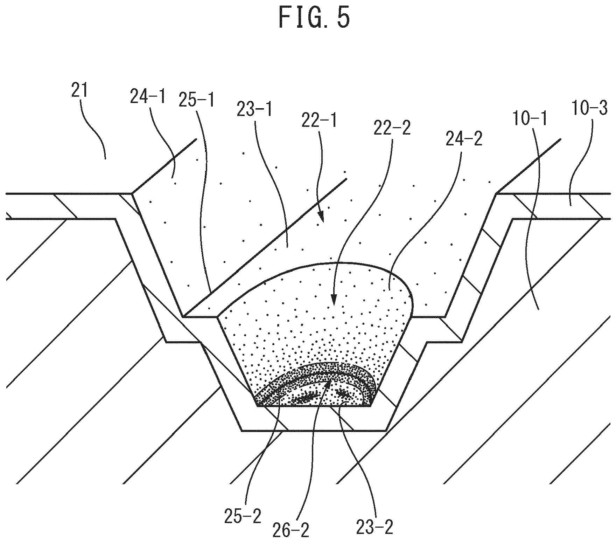

Fig. 2 is plan view of the skin side of the absorbent body 10 of the disposable diaper 1 of this embodiment. Fig. 3 is an end view of a cross section of the absorbent body 10 of Fig. 2 along line III-III, and Fig. 4 is a partial magnified view of a lattice-like first embossed section PI of the absorbent body shown in Fig. 2. Fig. 5 is a partial magnified view of a second embossed section P2 serving as curving origin (hinge) in the absorbent body shown in Fig. 2.

Referring to Fig. 2, the absorbent body 10 has a lengthwise direction L and a widthwise direction W perpendicular to the lengthwise direction L, the top end in Fig. 2 being the abdominal side and the bottom end being the back side, and while it is an essentially narrow rectangular shape at the center section in the lengthwise direction L, the location of narrowest width is nearer the abdominal side than the center in the lengthwise direction L. The absorbent body 10 is at the center section in the lengthwise direction L and the widthwise direction W, and it has a lattice-like first embossed section P1, and a pair of left and right second embossed sections P2 on both the left and right sides in the widthwise direction W of the absorbent body 10, extending in the lengthwise direction and serving as curving origins (hinges) for the absorbent body.

Referring to the end view of Fig. 3, the absorbent body 10 includes an absorbent core 10-1 and a core wrap 10-2 covering it, the core wrap 10-2 including an upper core wrap 10-3 and a lower core wrap 10-4. The absorbent core 10-1 typically includes pulp fibers and a superabsorbent polymer (SAP), and the core wrap 10-2 is made of tissue. In the regions of the first embossed section PI and the second embossed sections P2, the absorbent body 10 has recesses 22 running from the surface 21 of the upper core wrap 10-3 up to the core wrap 10-2, in the thickness direction of the absorbent body.

Fig. 4 is a partial magnified perspective view of the absorbent body 10 shown in Fig. 2 where the lattice-like first embossed section PI has been cut along line IV-IV. As shown in Fig. 2, the lattice-like first embossed section PI is formed by a combination of curved groove-shaped embossed sections (recesses 22), the recesses 22 being formed of bottom part 23 and wall sections 24. The recesses 22 have colored regions, the coloration being indicated here as dots. In Fig. 4 and Fig. 5, the dots representing the colored regions are used schematically to represent the coloration concentrations in the colored regions (defined as the grayscale value of the color, for the purpose of the present invention).

In Fig. 4, the bottom part 23 of the recess 22 is the section forming the bottom face of the recess 22, the bottom face of the bottom part 23 forming a surface that is essentially parallel to the surface 21 of the upper core wrap 10-3. The wall sections 24 of the recess 22 are sections forming wall faces that rise from the edges of the bottom part 23 toward the surface 21 of the upper core wrap 10-3. The angle α by which the wall faces of the wall sections 24 rise from the bottom face of the bottom part 23 is approximately 60°, and the angle between each wall face of the wall sections 24 in the recess 22 and the bottom face of the bottom part 23 is approximately 120°.

In Fig. 4, a linear region delineated from the border line 25 forming the border between the bottom part 23 and each wall section 24, with width w₁ inside the bottom part 23 and the width w₂ inside the wall section 24, for a total width w₃, is defined as a first linear region 26, and linear regions delineated with widths w₃ that are the same as the width w₃ of the first linear region 26, adjacent to the first linear region 26 on the bottom part 23, are defined as second linear regions 27. Furthermore, linear regions delineated with widths w₃ that are the same as the width w₃ of the first linear region 26, adjacent to the first linear region 26 on the wall sections 24, are defined as third linear regions 28. The width w₁ in the bottom part 23 may be the same as or different from the width w₂ in the wall sections 24. The recesses 22 have blue colored regions, for example, that are represented by the dots, but the grayscale value of coloration of the blue in the first linear region 26, which is the linear region straddling the border line 25, is lower than the grayscale value of coloration of the blue in the second linear region 27 and the third linear region 28. For this embodiment, the linear regions adjacent to the first linear region that are linear regions with higher grayscale values of coloration than the grayscale value of coloration of the first linear region (especially the linear region in the bottom part) are actually present in a wider width than the width of the first linear region. This is preferred because it will accentuate the presence of the first linear region that includes the border line between the bottom part and the wall section of the recess and has a low grayscale value of coloration. For this embodiment, the first linear region 26 is present straddling the border line 25, but it may instead be present only on the bottom part 23 side of the border line 25 and including the border line 25, and absent from the wall section 24 side. According to the invention, the colored regions in the recesses 22 may be only in the bottom part 23, or they may be present in both the bottom part 23 and the wall sections 24.

In Fig. 4, the colored regions are present not only in the first linear region 26 straddling the border line 25 between the bottom part 23 and the wall sections 24, but also in other regions of the bottom part 23 and the wall sections. In the regions of the bottom part 23 other than the first linear region 26, the grayscale value of coloration is generally higher than the first linear region 26, but there are also colored sections and non-colored sections, and the grayscale value of coloration is not uniform (it varies), while the grayscale value of coloration is also inconsistent even in the colored sections.

The grayscale value of coloration increases at the wall sections 24 as they recede from the border line 25 to the surface 21 side of the upper core wrap 10-3, producing a gradation in the grayscale value of coloration. With such gradation, the embossed sections appear three-dimensional and the embossed sections can be more clearly seen.

For this embodiment, the grayscale value of coloration in the first linear region may be a lower grayscale value of coloration (average value) than any region other than the first linear region having the same area as the first linear region.

According to the invention, however, the manner of coloration in the regions of the bottom part 23 or wall sections 24 other than the first linear region 26 is not limited to this form.

For this embodiment, the bottom parts 23 of the recesses 22 are flat and essentially parallel with the flat section formed by the lengthwise direction and the widthwise direction (referred to here as the "horizontal plane"), but according to the invention they do not necessarily need to be completely flat, and only need to be approximately flat, and constructed with surfaces (including curved surfaces) forming angles of no greater than 45° or no greater than 30° with respect to the horizontal plane. At the border lines between the bottom part and the wall sections, on the other hand, the wall sections have surfaces (wall faces) that rise in the thickness direction of the absorbent body toward the surface side of the upper core wrap, with respect to the surface of the bottom part, and the wall sections and bottom part are said to have curvature at the border lines. The angles of curvature of the wall sections and the bottom part at their border lines are preferably about 30° or greater and more preferably 45° or greater, and may be 60° or greater or even 90°. Also, they are preferably constructed with surfaces that rise at an angle of 45° or greater, and more preferably 50° or greater or 55° or greater (including curved surfaces) with respect to the horizontal plane, from the border lines between the bottom part and the wall sections. For example, in a recess where the bottom part is constructed so as to include a flat section or curved surface forming an angle of 30° with the horizontal (the bottom part will generally have a shallow bowl or saucer shape, but it may be constructed of a combination of a flat section or curved surface that forms an angle of 30° with the horizontal, and a flat section parallel to the horizontal), and the flat section or curved surface forming the angle of 30° is adjacent to the wall sections that rise at an angle of 90° with respect to the horizontal, and the curving angle is 60° at the border lines between the bottom part and the wall sections, it is possible to lower the grayscale values of coloration in the linear regions including the border lines between the bottom part and the wall sections.

Fig. 5 is a partial magnified perspective view of a second embossed section P2 serving as a curving origin (hinge) in the absorbent body 10 shown in Fig. 2. The second embossed section P2 is formed as a groove overall as shown in Fig. 3, but it also has a structure with a circular embossed section, rhomboid embossed section or oblong embossed section further formed in the groove. Fig. 5 is a partial magnified perspective view near a circular embossed section inside it.

Referring to Fig. 5, the depressed recess is formed in the upper core wrap 10-3 and the absorbent core 10-1, from the surface 21 of the upper core wrap 10-3 toward the absorbent core 10-1, but here the recess is a two-level embossed section. The first level of the embossed section (recess 22-1) is a groove, having a first-level flat bottom part 23-1 in the depthwise direction of the recess from the surface 21 of the upper core wrap 10-3, and wall sections 24-1. The first-level recess 22-1 is colored in both the bottom part 23-1 or the wall sections 24-1, but the grayscale value of coloration is a higher grayscale value of coloration than the grayscale value of coloration in the second-level recess 22-2, or else it is essentially non-colored. According to the invention, incidentally, the first-level recess may also be in a form that is completely without coloration. From the first-level bottom part 23-1, the second-level embossed section (recess 22-2) is additionally formed in the depthwise direction of the recess, the second-level recess 22-2 being a circular embossed section. The second-level recess 22-2 consists of a bottom part 23-2 and a wall section 24-2. The second-level recess 22-2 has colored regions represented by dots, the grayscale value of coloration being lower in the linear region 26-2 that includes the border line 25-2 between the bottom part 23-2 and the wall section 24-2, than in the adjacent regions on both sides. The aspect of the grayscale value of coloration in the second-level recess 22-2 in Fig. 5 is the same as the aspect of the grayscale value of coloration in the recess 22 of Fig. 4, and it will not be explained again here.

Incidentally, according to the invention, when the recess has two or more levels of bottom parts, the preferred design for the recess in terms of production is for a colored region to be present at least in the bottom part at the deepest location of the recess, with the grayscale value of coloration in the linear region including the border line between the bottom part and the wall section that rises from the bottom part being lower than the grayscale value of coloration in the adjacent regions.

In each of the second embossed sections P2, rhomboid embossed sections and oblong embossed sections are consecutively arranged as second-level recesses similar to the circular embossed recesses 22-2, inside the first-level recess 22-1 forming the grooves, and since all of them are colored, the colored regions of the circular embossed sections, rhomboid embossed sections and oblong embossed sections can be recognized as groove recesses when viewed from the skin side of the absorbent body (absorbent article).

With coloration of the embossed sections as explained with reference to Figs. 2 to 5, the colorations of the linear regions 26, 26-2 including the border lines 25, 25-2 between the bottom parts 23, 23-2 and wall sections 24, 24-2 allow the shapes of the embossed sections to be recognized, while the coloration of the bottom parts are non-uniform, so that the embossed sections appear undulating and a more natural visual effect is provided for the embossed sections. When the embossed sections are colored at a uniform density, the colored pattern becomes highly discernible, making it contrived and causing the embossed sections to appear unnatural.

Thus, according to the invention, coloration of the wall sections is not essential. It is also not essential for the wall sections of the recesses to have gradation in which the grayscale values of coloration increase as they recede from the linear regions including the border lines between the bottom parts and the wall sections. According to the invention, however, the wall sections of the recesses have colored regions, and it is preferred for the colored regions at the wall sections to have gradation in which the grayscale values of coloration increase as they recede from the linear regions including the border lines between the bottom parts and the wall sections. By having such colored regions at the wall sections, the user can more reliably recognize the presence of the embossed sections, and the shadow effect of the gradation can provide a feeling of assurance by creating a visual image of an integral state following firm embossed section shapes.

According to the invention, it is usually visibly clear by viewing a color photograph of the recesses, whether or not the linear regions including the border lines between the bottom parts and wall sections of the recesses include linear regions with lower grayscale values of coloration than the regions on both sides. If necessary, however, the grayscale values of coloration in regions of a recess such as shown in Fig. 4 and Fig. 5 can be compared by taking a photographic image of the region including the embossed section using a photographing apparatus (for example, a digital microscope VHX2000 by Keyence Corp.), and using image processing software to convert the photographic image data to grayscale color, and determining the grayscale value of coloration for each prescribed region on a line running through the recess.

According to the invention, the grayscale value of coloration is an index for evaluation of the concentration of the coloring agent in the recess, but the grayscale value of coloration is not the grayscale value for "coloration in the strict sense" as determined by the difference in the color after coloration of the base sheet compared to the color before coloration. According to the invention, the grayscale value of coloration represents the density of color in the recess after coloration, and represents the density of the color in the colored regions and the non-colored regions, obtained by combining the original color of the base sheet with the color of (additional) coloration by a coloring agent in the recess. According to the invention, therefore, the non-colored regions also have a grayscale value of coloration, and the original color of the base sheet (approximately white in most cases) is the grayscale value of coloration of the non-colored regions.

A low grayscale value of coloration according to the invention means, for the grayscale value of a given coloration, a small grayscale value of coloration where black is represented as 0 and white is represented as a positive value, and it corresponds to a low L* value (luminance) in the color chart system. The grayscale value of coloration may also be expressed with white as 0 and black as a positive value, but according to the invention the grayscale value of coloration used assumes black as 0 and white as (any) positive value. The grayscale value (maximum) of a substitute color may be 100, or 256 (tone representation with 8 bits), for example. Incidentally, the L* value (luminance) in the color chart system usually assigns 0 to black and 100 to white. The coloration is a chromatic color other than white. Coloration of the recess formed by embossing will usually be a single color. The grayscale value of coloration can still be evaluated even if the colored region has multiple colors, as it is sufficient if the relationship of the invention is satisfied for the grayscale value of coloration.

When a photographic image is taken, and a top sheet or the like is present further toward the skin side than the core wrap with the colored regions, it is preferred to measure the grayscale value of coloration by photographing the colored regions from the skin side including the top sheet, but since the evaluation is based on the relative density of coloration, the visibility of the colored regions through the top sheet may be separately confirmed after removing the members further toward the skin side than the core wrap with the colored regions, such as the top sheet, photographing the colored regions while the base sheet with the colored regions is exposed and measuring the grayscale value of coloration, and making a (relative) comparison with the grayscale value of coloration in the prescribed regions.

In a photographic image taken from directly above the recess, the border lines between the bottom part and the wall sections of the recess (hereunder also referred to simply as "border lines") may be established by comparison with an image under diagonal lighting of the same recess, for example. By determining the average value of the grayscale values of coloration of each region among the regions delineated at each fixed width from the border line, from the curve (graph) of the grayscale value of coloration on a line transecting the recess, it is possible to obtain a grayscale value of coloration in each region as a function of distance from the border line. In addition, the average is determined for the grayscale value of coloration in each region as a function of distance from the border line, measured at 5 or more arbitrary locations of the prescribed recesses or emboss pattern to be measured, and determined as described above, and is recorded as the grayscale value of coloration (average value) for the prescribed recesses or emboss pattern to be measured. The fixed width by which each region is delineated for measurement may be as desired so long as it is possible to discern the change in grayscale value of coloration on the line that is measured. For a line having a prescribed spacing from the border line, the width of the bottom part of the recess (for example, w₀ in Fig. 4) is determined in the direction perpendicular to the border line, and the width defined as every 5%, or every 10% or 15%, for example, with 100% as the width of the bottom part. The measured width may be even smaller if the concentration difference is large. In this manner it is possible to know whether or not the condition has been satisfied that the grayscale value of coloration in the linear region with a certain width including the border line is lower than the grayscale value of coloration in the linear regions of the same width on both sides. The widths of the linear regions may be as desired, as it is sufficient if the condition for the relationship of the grayscale value of coloration is satisfied in any linear region including the border line. Incidentally, it should be mentioned that the grayscale value of coloration and the L* value (luminance) in the color chart system are correlated, and the grayscale value of coloration of the invention can also be evaluated by the L* value in the color chart system.

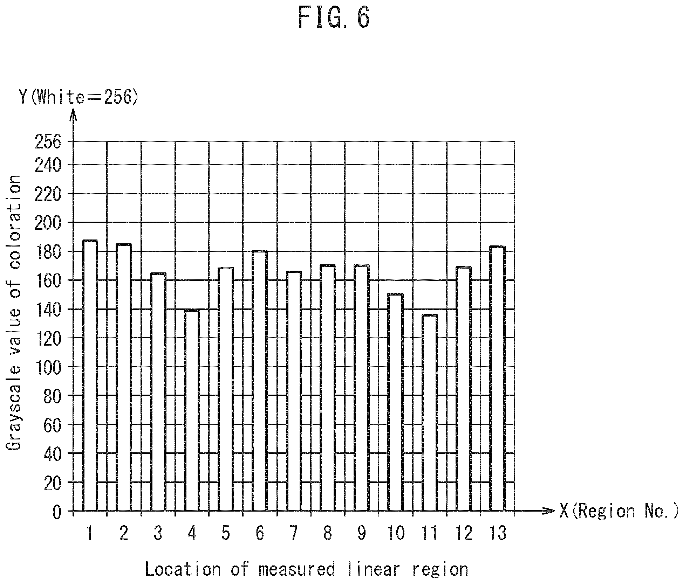

Fig. 6 shows an example of measuring the grayscale value of coloration in the widthwise direction of a groove-shaped recess of a lattice-like embossed section as shown in Fig. 4. The grayscale value of coloration of the groove-shaped recess 22 shown in Fig. 4 was measured along a line in a direction perpendicular to the border line 25 between the bottom part 23 and wall section 24 of the recess 22, and the average value of the grayscale value of coloration for each region of fixed width (10 pixel, approximately 0.085 mm) on the measured line was calculated. Similar measurement and calculation were also performed for a total of 5 locations from the same groove-shaped recess of the lattice-like embossed section, along a line in a direction perpendicular to the border line, and the obtained grayscale values of coloration of the respective regions at the 5 locations were averaged to calculate the grayscale value of coloration (average value) at each region of fixed width at the same distance from the border line after matching the positions of the border line. In Fig. 6, the abscissa represents the positions of each of the regions of fixed width on the lines measured, as the region numbers (No.). The border line between the bottom part and the wall section of the groove-shaped recess of the lattice-like embossed section was confirmed to be in the regions of region No. 4 and No. 11 on the abscissa. The width of each region was approximately 0.085 mm (10 pixels). The ordinate is the grayscale value of coloration for each region of fixed width (0 being black and 256 being white).

According to Fig. 6, it is seen that the grayscale value of coloration was lower in region No. 4 which included the border line 25 between the bottom part 23 and the wall section 24, than in either region No. 3 or No. 5 flanking both sides of region No. 4, and likewise, the grayscale value of coloration was lower in region No. 11 which included the border line 25 between the bottom part 23 and wall section 24, than in either region No. 10 or No. 12 flanking both sides of region No. 11. It is also seen that the grayscale value of coloration at the center section of the bottom part between regions No. 4 and No. 11 including the border line 25 was not uniform, with high and low variation. In addition, a gradation can be seen in which the grayscale value of coloration increased on the wall sections (No. 3 to No. 1 and No. 11 to No. 13), on the outer sides of regions No. 4 and No. 11 which included the border line 25, as they receded from regions No. 4 and No. 11 that included the border line 25.

According to the invention, the width of the linear region of a certain width including the border line between the bottom part and the wall section of the recess, which has a grayscale value of coloration that is lower than the grayscale value of coloration in the linear regions of the same width on both sides, is not particularly restricted, but since a colored region is usually present at the bottom part of the recess, a restriction therefore exists based on the dimensions of the bottom part of the recess. The width of the colored region at the bottom part of the recess cannot be larger than 1/3 of the dimension of the bottom part of the recess in the direction perpendicular to the border line (the circular diameter in the case of a circular recess). The width of a colored region with a low grayscale value of coloration on the bottom part and wall section of the recess is preferably no greater than 1/4 or no greater than 1/5, and may even be no greater than 1/10 or no greater than 1/20, of the dimension of the bottom part of the recess. Also, the lower limit for the width of the colored region is not restricted and may be, for example, at least 1/50 of the dimension of the bottom part of the recess. On the other hand, the widths of the regions with high grayscale values of coloration adjacent to both sides of the colored region with a low grayscale value of coloration may be the same as or greater than the width of the colored region with a low grayscale value of coloration, but preferably they are at least 1/20, and more preferably at least 1/10 or at least 1/5, of the dimension of the bottom part of the recess. Incidentally, the regions with high grayscale values of coloration on the outer sides of the colored region with low grayscale value of coloration are not only the wall sections of the recesses, but may also be the outer sides of the wall sections. Thus, the width of the colored regions with low grayscale values of coloration occupying the wall sections of the recesses are at maximum the widths of the wall sections.

According to the invention, the grayscale value is represented with a numerical value of 0 for black and a positive numerical value for white (which may be any value such as 100 or 256, for example), as in the example of Fig. 6. In order to match the high/low variation in the grayscale value of coloration with the high/low variation in the coloration density, it is reasonable to determine the difference between the grayscale value of coloration for white (for example, 100%) and the grayscale value of coloration in the colored region (y%) ((100 - y)%), and to use the value of the difference in the grayscale values of coloration to express the "coloration concentration". For the coloration concentration based on this definition ((100 - y)%), the coloration concentration in the linear region must be higher than the coloration concentration in the regions of the same width on both sides of the linear region, and if the coloration concentration in the linear region is a reference value of 100%, the difference between the coloration concentration in the linear region and the coloration concentration in the regions on both sides is preferably 10% or greater, more preferably 15% or greater, even more preferably 20% or greater, or even 30% or greater or 40% or greater. Preferably, the difference in coloration concentration from comparison of the coloration concentrations is ignored at below a certain proportion (for example, ≤5%), as being not significantly different.

According to the invention, when several types of recesses (embossed sections) have been formed in the absorbent article, the method of coloration of the invention may be applied only in specific types of recesses (embossed sections) among the different types of recesses (embossed sections). When the individual embossed sections (recesses) are considered, and the length of the border line between the bottom part and the wall section of each recess is not very long, as with circular embossed sections, rhomboid embossed sections or oblong embossed sections, the grayscale value of coloration is measured in the direction perpendicular to the border line, selecting 5 locations from among the type of recesses (embossed sections) being considered, and the average value is calculated. In order to evaluate the recesses as grooves including the circular embossed sections, rhomboid embossed sections and oblong embossed sections, 5 arbitrary embossed sections are selected from among the embossed sections, the grayscale values of coloration are measured by the method described above, and their average value is calculated. When the length of the border line between the bottom part and the wall section is very long, as with a lattice-like embossed section, 5 arbitrary locations of the embossed section are selected, the grayscale values of coloration are measured by the method described above and the average value is recorded as the measured value for the grayscale value of coloration of the lattice-like embossed section. The recesses (embossed sections) in which the grayscale value of coloration satisfies the condition of the invention preferably constitute 20% or greater, 30% or greater or 50% or greater of the recesses (embossed sections) forming the absorbent body, based on the area of the recesses (embossed sections).

For this embodiment, the shapes of the embossed sections (recesses) are circular, rhomboid, oblong or lattice-like, but the embossed sections (recesses) are obviously not limited to these shapes and may be various different types. For example, they may be not only regular shapes such as square or triangular, but also designs or patterns with freely selected shapes, such as flowers.

The construction of the absorbent body 10 of this embodiment includes an absorbent core 10-1 and a core wrap 10-2 that covers it. For this embodiment, the absorbent core 10-1 contains pulp fiber and a superabsorbent polymer (SAP) and the core wrap 10-2 comprises tissue paper, but according to the invention the constructions used for the absorbent core and the core wrap covering it may be any known ones.

Examples of components for the absorbent core 10-1 generally include hydrophilic fibers, and more specifically, cellulose such as ground pulp or cotton; regenerated cellulose such as rayon or fibril rayon; semi-synthetic cellulose such as acetate or triacetate; filamentous polymers; thermoplastic hydrophobic chemical fibers; and hydrophilicized thermoplastic hydrophobic chemical fibers; as well as any combinations of the foregoing. The component of the absorbent core may also be, for example, granules comprising a superabsorbent polymer, such as sodium acrylate copolymer. However, the absorbent core most preferably contains ground pulp and a superabsorbent polymer (SAP) from the viewpoint of absorption performance.

The core wrap 10-2 is not particularly restricted so long as it has sufficient liquid permeability to allow permeation of fluids such as body fluids discharged from the human body and has a sufficient barrier property to prevent permeation of the component of the absorbent core enclosed therein, and examples include sheet-like fiber structures such as woven fabrics, nonwoven fabrics and tissues. The constituent material may be natural fibers, chemical fibers or the like. The core wrap is preferably a tissue that allows increase in the coloration density (lowering of the grayscale value of coloration).

### (Method of forming colored regions)

Methods for coloring the recesses of the absorbent body are known and any may be employed, such as a method of printing by an ink-jet method after formation of the recesses, but for the invention a method of coloring during formation of the recesses by embossing is preferred. Specifically, it is preferred to employ a printing method in which ink is set on the raised sections (the sections that are to form the recesses, or the sections constituting the printing plates) of the embossing roll during embossing, or a method in which the absorbent body is coated with a pressure sensitive coloring agent and pressed by embossing to develop the color of the pressure sensitive coloring agent.

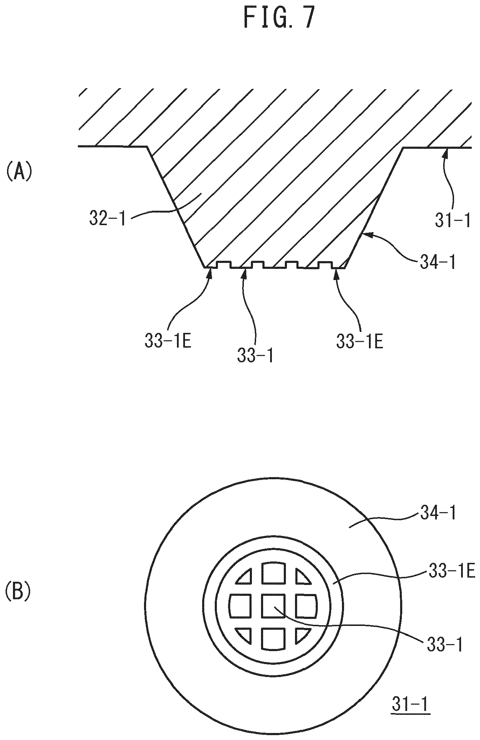

During formation of the recesses by embossing, in order to lower the grayscale values of coloration in the linear regions that include the border lines between the bottom parts and wall sections of the recesses, a method may be used in which completely flat surfaces (printing density: 100%) are formed at the tip sections of the raised sections of the embossing roll (the plate surface sections) in the linear regions including the sections that are the border lines between the bottom parts and wall sections of the recesses formed by embossing, and the area density (printing density) at the flat surfaces where the ink has been set is reduced in the other sections, or the heights of the flat surfaces where the ink is to be set are reduced at the other sections, compared to the linear regions, to make them less likely to contact with the substrate that is to be printed. Fig. 7, for example, is a magnified cross-sectional view and a plan view of a raised section 32-1 formed in an embossing roll for formation of a recess 22 as the embossed section shown in Fig. 4. The raised section 32-1 is a raised section with respect to flat sections 31-1 that contact with the flat surface 21 of the upper core wrap. The raised section 32-1 has wall faces 34-1 that extend from the flat sections 31-1, the wall faces 34-1 being surfaces that are to form the wall sections 24 of the recess 22. The raised section 32-1 has at its tip an approximately flat surface 33-1 that is to form the bottom part 23 of the recess 22. Fig. 7(A) shows a cross-section of the raised section, and Fig. 7(B) is a plan view as seen from the leading edge of the raised section. In the approximately flat surface 33-1 that is to form the bottom part 23 of the recess 22, outer perimeter regions 33-IE with prescribed widths running along the border lines with the wall faces 34-1 (that is, the outer perimeters of the surface 33-1) are flat, while at the region on the inside of the outer perimeter regions 33-IE, flat surfaces of the same height as the flat surface of the outer perimeter region 33-IE are finely fragmented, the area density of the flat sections being smaller than the area density of the outer perimeter region 33-IE. In particular, the inside circumferential region adjacent to the flat surface of each outer perimeter region 33-IE is formed lower than the flat surface of the outer perimeter region 33-1E. When embossing is carried out using an embossing roll having such raised sections, with ink set on the flat surfaces 33-1 at the tips of the raised sections, the grayscale value of coloration (average value) can be lowered at the sections in contact with the outer perimeter regions 33-1E, while the grayscale value of coloration (average value) can be increased in the region further inward than the outer perimeter regions 33-1E.

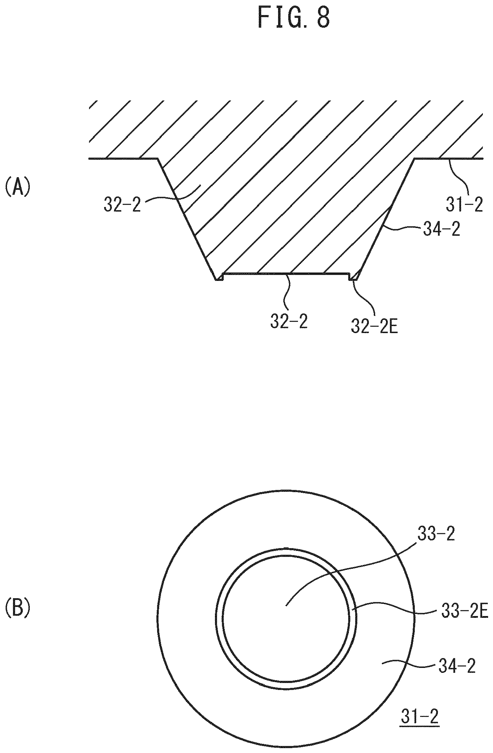

Furthermore, when using a method of coating the absorbent body with a pressure sensitive coloring agent and embossing, it may be preferred to employ a method in which the heights of the locations that include the border lines between the bottom parts and wall sections of the recesses are increased compared to the other locations (increased height being in the direction toward the tips of the raised sections), at the flat pressing parts (plate surfaces) of the tips of the raised sections of the embossing roll, to increase the pressing force and lower the grayscale value of coloration at those locations. Fig. 8 is a magnified cross-sectional view and a plan view of a raised section 32-2 formed in a different embossing roll for formation of a recess 22 as the embossed section shown in Fig. 4. The raised section 32-2 is similar to the raised section 32-1 of the embossing roll in Fig. 7, and it has flat sections 31-2 that are to contact with the flat surface 21 of the upper core wrap, wall faces 34-2 that are to form the wall sections 24 of the recess 22, and an approximately flat surface 33-2 that is to form the bottom part 23 of the recess 22. However, the outer perimeter region 33-2E of prescribed width running along the border line with the wall faces 34-2 (that is, the outer perimeter of the surface 33-2) protrudes outward with respect to the approximately flat surface 33-2 that is to form the bottom part 23. When an embossed section is colored using an embossing roll having such raised sections and a pressure sensitive coloring agent, the pressing force can be increased at the outer perimeter region 33-2E compared to the depressed section of the approximately flat surface 33-2 that is to form the bottom part 23, and although the pressing force of the outer perimeter region 33-2E is lower at the depressed section of the approximately flat surface 33-2 that is to form the bottom part 23 (the regions other than the outer perimeter region 33-2E), sufficient pressing force is obtained for color development of the pressure sensitive coloring agent. As a result, the pressing force is increased in the outer perimeter region 33-2E compared to the depressed section of the approximately flat surface 33-2 that is to form the bottom part 23, and the grayscale value of coloration can be reduced. Incidentally, although the outer perimeter region 33-2E has a prescribed width in Fig. 7, the same effect can be obtained even when the tip of the outer perimeter region 33-2E is a linear protrusion without width. Furthermore, even when a pressure sensitive coloring agent is used, it is possible to use an embossing roll having a pattern of the raised sections 32-1, of which one is shown in Fig. 7, and since in such a case the area density on which the pressing force is applied from the raised sections will differ between the outer perimeter region and the inner region, it will be appreciated that a difference in grayscale values of coloration can be provided between the outer perimeter region and the inner region.

When a method of coating a pressure sensitive coloring agent onto the absorbent body and embossing is used to form colored regions in the recesses, it is preferred because it will be possible to obtain coloration having a grayscale value of coloration reflecting irregularities or differences in hardness due to the fiber material or particles composing the base sheet on the surface of the base sheet side, even when the recess-formed surface on the embossing roll side is flat, and therefore coloration of the recesses will be more natural.

During formation of the recesses by embossing, the method used for coloring of the wall sections of the recesses may be a method of setting ink also on the locations that are to form the wall sections of the raised sections of the embossing roll, or a method of coating a pressure sensitive coloring agent onto the absorbent body, changing the angles of inclination or the shapes at the locations corresponding to the wall sections of the recesses that are formed at the raised sections of the embossing roll, and producing color by developing the color of the pressure sensitive coloring agent even at the wall sections of the recesses. The method of forming gradation in the coloration of the wall sections of the recesses may also be similar. In other words, it may be a method in which the locations that are to form the wall sections at the raised sections (printing plate surfaces) of the embossing roll are formed by dots, and the density of the dots decreases while receding from the border lines between the wall sections and the bottom part in the depthwise direction of the recesses, or a method in which, for coloration using a pressure-sensitive adhesive, the pressure applied to the substrate (and pressure sensitive coloring agent) to be colored, on the wall sections of the recesses, decreases from the border lines between the recesses and the wall sections toward the surface of the base sheet, in the direction receding from the border lines between the wall sections and the bottom parts.

### (Pressure sensitive coloring agent)

A microencapsulated pressure sensitive coloring agent to be used for the preferred embodiment will now be described.

Microcapsules confining a color-forming agent (coloring-forming microcapsules) may be used as the pressure sensitive coloring agent. The color-forming microcapsules are microparticles with sizes of about 1 to 200 µm and preferably about 3 to 20 µm, comprising a liquid or solid core substance (containing a color former) covered with a film substance. Microcapsules containing a color former and developing agent and methods for their production are known (see Japanese Unexamined Patent Publication No. 2007-132756 and Japanese Unexamined Patent Publication No. 2010-173087, for example).

When the color-forming microcapsules are subjected to pressure, they disintegrate and release the color former. Conditions under which the color-forming microcapsules disintegrate are preferably disintegration at a pressure of 2.5 to 300 MPa and especially 50 to 150 MPa, for example. Heating may also be carried out along with the pressure during embossing.

A color former is a substance (agent) that reacts with a developing agent to express a color. Color formers that may be used include publicly known electron-donating color formers commonly used for pressure-sensitive paper and the like, and examples include blue color formers such as crystal violet lactone, red color formers such as 2-chloro-3-methyl-6-diethylaminofluorane and green color formers such as 2-dibenzylamino-6-diethylaminofluorane.

The developing agent is a substance (agent) that can react with a color former (a color former released from microcapsules) to express a color. The developing agent used may be a known electron-accepting developing agent, and examples include phenol resin-based compounds, salicylic acid-based metal chlorides, salicylic acid resin-based metal oxides and solid acid-based compounds.

After the absorbent body has been coated with the color-forming microcapsules and developing agent, it may be pressed with an embossing roll to form the embossed sections (compressed recesses). Alternatively, after the absorbent body has been coated with the color-forming microcapsules, it may be pressed with an embossing roll to form the embossed sections (compressed recesses), and then coated with the developing agent. The pressure sensitive coloring agent may be coated on the surface of either the non-absorbent core side or absorbent core side of the upper core wrap.

In the disposable diaper 1 of the embodiment described with reference to Figs. 1 to 8, the liquid-permeable base sheet on which the colored region is to be formed in the recesses 22 is the core wrap 10-2 of the absorbent body 10., According to a reference example, the base sheet on which the recesses and colored regions are to be formed may be, instead of the core wrap, the top sheet, or another liquid-permeable sheet (not shown) between the absorbent core and the top sheet. When the base sheet is the top sheet, for example, after covering the absorbent core with the core wrap to form the absorbent body, and laminating the top sheet on the absorbent body, embossing may be carried out to form the first embossed sections as curving origins and the lattice-like second embossed sections, as embossed sections (recesses) running from the top sheet up to the absorbent core, and during the embossing of the top sheet, it is possible to form colored regions in the recesses that are to be formed in the top sheet and absorbent body, using a coloring agent by the method of coloring the core wrap as described above.

### (Other embodiments)

It is also possible to form colored regions in both the core wrap and the top sheet.

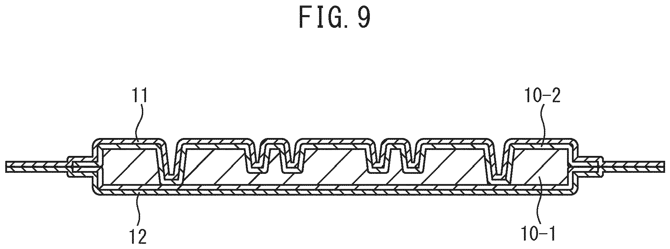

Fig. 9 shows an example of an absorbent article having recesses formed running from the top sheet 11 to the absorbent core 10-1, as a cross-sectional end view of the center section of a disposable diaper in the lengthwise direction. In Fig. 9, 10-1 is the absorbent core, 10-2 is the core wrap, 11 is the top sheet and 12 is the back sheet.

As a separate embodiment having the same construction as the disposable diaper 1 according to the embodiment described with reference to Figs. 1 to 9, second colored regions may be formed in the sections coinciding with the recesses (first embossed sections and second embossed sections) of the lower core wrap of the absorbent body. The second colored regions formed in the lower core wrap may have the same characteristics as the colored regions formed in the upper core wrap (where the grayscale value of coloration in the linear regions including the border lines between the bottom parts and wall sections of the recesses are lower than in the linear regions on both sides), but this is not essential. In addition, coloration of the lower core wrap may be from the absorbent core side of the lower core wrap, from the opposite non-skin side, or inside the lower core wrap.

Moreover, although a disposable diaper was described as an embodiment of the absorbent article of the invention, the invention may be applied not only to a disposable diaper but also to an absorbent article such as an incontinence pad or sanitary napkin.

### REFERENCE SIGNS LIST

- 1: Disposable diaper
- 10: Absorbent body
- 10-1: Absorbent core
- 10-2: Core wrap
- 10-3: Upper core wrap
- 10-4: Lower core wrap
- 11: Top sheet
- 12: Back sheet
- P1: First embossed section (lattice-like embossed section)
- P2: Second embossed section (embossed section serving as curving origin)
- 21: Surface of absorbent body (upper core wrap)
- 22: Recess
- 22-1: First-level recess
- 22-2: Second-level recess
- 23: Bottom part
- 23-1: First-level bottom part
- 23-2: Second-level bottom part
- 24: Wall section
- 24-1: First-level wall section
- 24-2: Second-level wall section
- 25: Border line between bottom part and wall section
- 25-2: Border line between second-level bottom part and wall section
- 26: First linear region
- 26-2: Second-level first linear region
- 27: Second linear region
- 28: Third linear region
- 31-1, 31-2: Flat section contacting with surface of upper core wrap of embossing roll
- 32-1, 32-2: Raised section of embossing roll
- 33-1, 33-2: Surface of embossing roll to form bottom part of recess (embossed section)
- 33-1E, 33-2E: Outer perimeter region
- 34-1, 34-2: Wall section of embossing roll

## Claims

1. An absorbent article (1) comprising an absorbent core (10-1) and a liquid-permeable base sheet on the skin side of the absorbent core, and having a thickness direction, wherein
the base sheet and the absorbent core (10-1) have recesses (22) extending in the thickness direction from the surface of the skin side of the base sheet up to the absorbent core,
the recesses (22) each have a bottom part (23) and wall sections (24) that rise from the bottom part in a direction toward the surface side of the skin side,
the base sheet has, in the recesses (22), colored regions that are visible from the skin side, and
the grayscale value (where black is represented by 0 and white is represented by any positive value, same hereunder) of the coloration of each linear region (26) that includes the border line (25) between the bottom part (23) and the wall section (24) and is delineated by a prescribed width is lower than the grayscale value of the coloration in each of the regions (27, 28) delineated by the same width as the prescribed width of the linear region, on both sides adjacent to the linear region (26),
wherein the absorbent article has a core wrap (10-2) covering the absorbent core (10-1), the core wrap has an upper core wrap (10-3) on the skin side of the absorbent core, the base sheet is the upper core wrap (10-3), and at the recesses (22), the surface of the skin side or the absorbent core side of the upper core wrap (10-3) is colored with a pressure sensitive coloring agent.

2. The absorbent article according to claim 1, wherein the grayscale values of coloration in the regions other than the linear regions of the bottom parts (23) are not uniform.

3. The absorbent article according to claim 1 or 2, wherein the recesses (22) have colored regions in both the bottom parts (23) and the wall sections (24).

4. The absorbent article according to claim 3, wherein the colored regions of the wall sections (24) have grayscale values of coloration that are higher as they recede from the linear regions in the thickness direction.

5. The absorbent article according to any one of claims 1 to 4, wherein the core wrap (10-2) has a lower core wrap (10-4) on the non-skin side of the absorbent core (10-1) and has a second colored region that coincides with the recesses (22), on the surface of the lower core wrap, and the second colored region is visible from the non-skin side.

6. The absorbent article according to any one of claims 1 to 5, wherein the core wrap (10-2) is tissue.

## Patentansprüche

1. Absorbierender Artikel (1), der einen Saugkern (10-1) und eine flüssigkeitsdurchlässige Grundlage auf der Hautseite des Saugkerns umfasst und eine Dickenrichtung aufweist, wobei
die Grundlage und der Saugkern (10-1) Aussparungen (22) aufweisen, die sich in der Dickenrichtung von der Oberfläche der Hautseite der Grundlage bis zum Saugkern erstrecken,
die Aussparungen (22) jeweils ein Bodenteil (23) und Wandabschnitte (24) aufweisen, die von dem Bodenteil in einer Richtung zu der Oberflächenseite der Hautseite aufragen,
die Grundlage in den Aussparungen (22) farbige Bereiche aufweist, die von der Hautseite sichtbar sind, und
der Grauwert (wobei schwarz durch 0 dargestellt wird und weiß durch jedweden positiven Wert dargestellt wird, gleich nachstehend) der Färbung von jedem linearen Bereich (26), der die Grenzlinie (25) zwischen dem Bodenpart (23) und dem Wandabschnitt (24) einschließt und durch eine vorgeschriebene Breite abgegrenzt ist, niedriger ist als der Grauwert der Färbung in jedem der Bereiche (27, 28), die durch die gleiche Breite wie die vorgeschriebene Breite des linearen Bereichs auf beiden Seiten benachbart zu dem linearen Bereich (26) abgegrenzt sind,
wobei der absorbierende Artikel eine Kernumhüllung (10-2) aufweist, die den Saugkern (10-1) abdeckt, wobei die Kernumhüllung eine obere Kernumhüllung (10-3) auf der Hautseite des Saugkerns aufweist, die Grundlage die obere Kernumhüllung (10-3) ist und an den Aussparungen (22) die Oberfläche der Hautseite oder der Saugkemseite der oberen Kernumhüllung (10-3) mit einem druckempfindlichen Farbstoff gefärbt ist.

2. Absorbierender Artikel nach Anspruch 1, wobei die Grauwerte der Färbung in den Bereichen, die nicht die linearen Bereiche der Grundteile (23) sind, nicht einheitlich sind.

3. Absorbierender Artikel nach Anspruch 1 oder 2, wobei die Aussparungen (22) farbige Bereiche in den Grundteilen (23) als auch den Wandabschnitten (24) aufweisen.

4. Absorbierender Artikel nach Anspruch 3, wobei die farbigen Bereiche der Wandabschnitte (24) Grauwerte der Färbung aufweisen, die mit Entfernung von den linearen Bereichen in der Dickenrichtung höher werden.

5. Absorbierender Artikel nach einem der Ansprüche 1 bis 4, wobei die Kernumhüllung (10-2) eine untere Kernumhüllung (10-4) auf der Nicht-Hautseite des Saugkerns (10-1) aufweist und einen zweiten farbigen Bereich, der mit den Aussparungen (22) zusammenfällt, auf der Oberfläche der unteren Kernumhüllung aufweist und der zweite farbige Bereich von der Nicht-Hautseite sichtbar ist.

6. Absorbierender Artikel nach einem der Ansprüche 1 bis 5, wobei die Kernumhüllung (10-2) Tissue-Papier ist.

## Revendications

1. Article absorbant (1) comportant une partie centrale absorbante (10-1) et une feuille de base perméable aux liquides sur le côté peau de la partie centrale absorbante, et ayant une direction allant dans le sens de l'épaisseur, dans lequel
la feuille de base et la partie centrale absorbante (10-1) ont des évidements (22) s'étendant dans la direction allant dans le sens de l'épaisseur depuis la surface du côté peau de la feuille de base jusqu'à la partie centrale absorbante,
les évidements (22) ont chacun une partie formant fond (23) et des sections formant paroi (24) qui s'élèvent depuis la partie formant fond dans une direction allant vers le côté surface du côté peau,
la feuille de base a, dans les évidements (22), des régions colorées qui sont visibles depuis le côté peau, et
la valeur d'échelle de gris (où le noir est représenté par 0 et le blanc est représenté par une valeur positive quelconque, de même ci-dessous) de la coloration de chaque région linéaire (26) qui comprend la frontière (25) entre la partie formant fond (23) et la section formant paroi (24) et qui est délimitée par une largeur prescrite est inférieure à la valeur d'échelle de gris de la coloration dans chacune des régions (27, 28) délimitées par la même largeur que la largeur prescrite de la région linéaire, des deux côtés de manière adjacente par rapport à la région linéaire (26),
dans lequel l'article absorbant a une enveloppe de partie centrale (10-2) recouvrant la partie centrale absorbante (10-1), l'enveloppe de partie centrale a une enveloppe de partie centrale supérieure (10-3) sur le côté peau de la partie centrale absorbante, la feuille de base est l'enveloppe de partie centrale supérieure (10-3), et au niveau des évidements (22), la surface du côté peau ou du côté partie centrale absorbante de l'enveloppe de partie centrale supérieure (10-3) est colorée au moyen d'un agent de coloration sensible à la pression.

2. Article absorbant selon la revendication 1, dans lequel les valeurs d'échelle de gris de coloration dans les régions autres que les régions linéaires des parties formant fond (23) ne sont pas uniformes.

3. Article absorbant selon la revendication 1 ou la revendication 2, dans lequel les évidements (22) ont des régions colorées à la fois dans les parties formant fond (23) et dans les sections formant paroi (24).

4. Article absorbant selon la revendication 3, dans lequel les régions colorées des sections formant paroi (24) ont des valeurs d'échelle de gris de coloration qui sont supérieures alors qu'elles s'éloignent des régions linéaires dans la direction allant dans le sens de l'épaisseur.

5. Article absorbant selon l'une quelconque des revendications 1 à 4, dans lequel l'enveloppe de partie centrale (10-2) a une enveloppe de partie centrale inférieure (10-4) sur le côté non-peau de la partie centrale absorbante (10-1) et a une deuxième région colorée qui coïncide avec les évidements (22), sur la surface de l'enveloppe de partie centrale inférieure, et la deuxième région colorée est visible depuis le côté non-peau.

6. Article absorbant selon l'une quelconque des revendications 1 à 5, dans lequel l'enveloppe de partie centrale (10-2) est un tissu ouaté.
